(19) Europäisches Patentamt
European Patent Office
Office européen des brevets

(11) Publication number: **0 246 709**
**A1**

# (12) EUROPEAN PATENT APPLICATION

(21) Application number: **87200940.2**

(22) Date of filing: **19.05.87**

(51) Int. Cl.⁴: **C12N 15/00** , C07H 21/02 , C07H 21/04 , C07K 15/06 , C12Q 1/68 , G01N 33/53 , G01N 33/577

(30) Priority: **20.05.86 NL 8601271**

(43) Date of publication of application: **25.11.87 Bulletin 87/48**

(84) Designated Contracting States: **BE DE FR GB LU NL**

(71) Applicant: **Stichting Katholieke Universiteit**
**Toernooiveld 1**
**NL-6525 ED Nijmegen(NL)**

(72) Inventor: **Van de Ven, Willem Jan Marie**
**Korhoenderveld 4**
**NL-5431 HH Cuyk(NL)**
Inventor: **Roebroek, Antonius Johannes Maria**
**De Bies 32**
**NL-6581 RJ Malden(NL)**
Inventor: **Schalken, Jacobus Antonius**
**Zwanenveld 75-22**
**NL-6538 TP Nijmegen(NL)**

(74) Representative: **Smulders, Theodorus A.H.J. et al**
**Vereenigde Octrooibureaux Nieuwe Parklaan 107**
**NL-2587 BP 's-Gravenhage(NL)**

## (54) Recombinant DNA and cDNA, mRNA, protein, antibodies, and a method of detecting tumor cells.

(57) This invention concerns reccmbinant DNA carrying the genetic information for a new protein called furin, as well as this protein proper and antibodies against the protein. The protein has been found to be, in particular, strongly expressed in specific types of tumors, such as non-small-cell lung tumors, mamma carcinomas, colon carcinomas, urogenital tumors en hematological malignities. Labelled RNA or DNA probes of the <u>fur</u> gene and antibodies against furin can be used for diagnostic purposes.

FIG.6

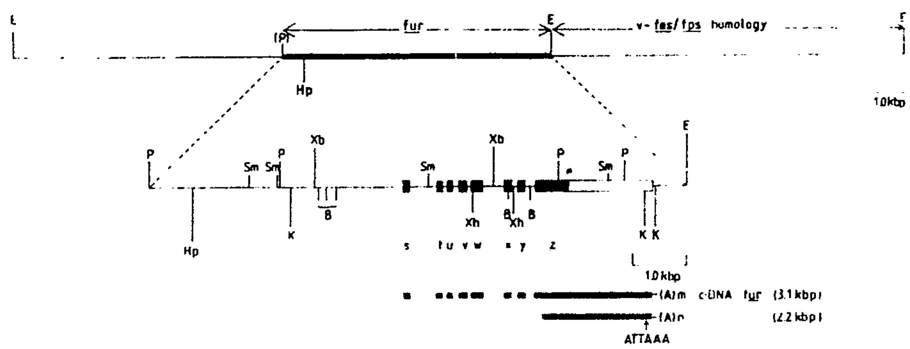


EP 0 246 709 A1

# Recombinant DNA and cDNA, mRNA, protein, antibodies, and a method of detecting tumor cells.

This invention is in the field of genetic engineering and relates in particular to the identification of a new gene having particular properties.

The new gene in question, which will be referred to herein as fur, is in the human and feline genome directly upstream of the fes/fps proto-oncogene.

Proto-oncogenes are cellular genes whose gene products are components of signal-transduction systems through which normal growth and differentiation of cells is regulated. In the case of transduction by a retrovirus they exhibit a transforming effect. These genes have been found to be prone to malign degeneration and, as such, they are indeed involved in the development of cancer. Indeed, in the latter case, they are referred to as oncogenes rather than proto-oncogenes. The degeneration of a proto-oncogene is often the result of a somatic mutation. This gives changes in the regulation of the expression of the gene and/or in the structure of the gene product, as also takes place upon retroviral mobilisation of the transforming potential of proto-oncogenes.

Some thirty proto-oncogenes are known at present, and indications have been obtained with regard to the possible function of some of them. The product of the proto-oncogene sis, for example, greatly resembles a growth factor occurring in blood platelets (platelet-derived growth factor). Products of other proto-oncogenes are greatly homologous to receptors to certain growth factors, such as, for example the epidermal growth factor receptor (c-erbB), the insulin receptor (c-ros), the receptor for CSF-1 (colony stimulating factor) (c-fms) and the glucocorticoid receptor (c-erbA). Some proto-oncogene products occur in cells of different tissue types, others are found in specialized cells only. The product of the fes/fps proto-oncogene, for example, appears to occur in blood cells of the myeloid series only.

It is generally assumed that proto-oncogene products will be of significance for diagnostics and possibly also therapy of all sorts of forms of cancer. To mention one example, the growth factor bombesin and the associated bombesin receptor are at present being used in the diagnosis of lung carcinoma. Lung tumors can roughly be classified in two categories, namely, the small-cell lung carcinomas (20-25%) and the non-small-cell lung carcinomas (75-80%). The above markers are only useable, however, for the identification and characterization of small-cell lung carcinomas. For non-small-cell lung carcinomas no good markers are available at present, although the epidermal growth factor receptor is being mentioned in this connection.

The fur gene was discovered by comparing inserts of certain cosmid clones with each other by means of Southern blot analysis. The cosmid clones in question were isolated from cosmid gene banks of human and feline DNA after hybridization with the v-fes DNA probe; v-fes being a viral oncogene occurring in the Gardner-Arnstein and the Snyder-Theilen strains of feline sarcoma virus. For details reference is made to the experimental part of this specification.

The transcript (messenger RNA of mRNA) of the fur gene or transcription unit is found to be about 4.5 kb long. The 4.5 kb fur mRNA was first discovered through Northern blot analysis of mRNA of tissue culture cells by means of fur genomic probes. After cloning fur-specific sequences in plasmids with an SP6 promotor (Melton et al., Nucleic Acids Research, 12, 7035-7056 (1984)) RNA transcripts of the fur gene could be synthesized in vitro.

fur cDNA was synthesized by means of reverse transcriptase, and isolated after cDNA cloning in λgt11, and hybridization with a fur genomic probe. For further details reference is again made to the experimental section of this specification.

On the ground of the computer analysis of the amino acid sequence derived from the fur cDNA, the translation product of the fur gene is found to be a protein having receptor characteristics. This protein will be referred to herein as furin.

Conventional antisera with a furin specificity could be prepared in rabbits by standard procedures. Monoclonal antisera could be prepared by the techniques of Kohler and Milstein (Nature 256, 495-497 (1978)). Fusion products of furin produced in E. coli K12 were used as antigene (Grayeb et al., EMBO J. 3, 2437-2442 (1984) and Molecular Cloning, A Laboratory Manual. T. Maniatis, E.F. Fritsch and J. Sambrook. Cold Spring Harbor Laboratory, 1982).

The present invention accordingly relates to the following products:

a. The fur gene or transcription unit. In men and cat, this gene is located immediately upstream of the fes/fps proto-oncogenes.

b. the 4.5 kb transcript (mRNA) of the fur gene.

c. the fur cDNA prepared by means of the fur mRNA.

d. The translation product of the fur gene, called furin.

e. Antisera having a furin specificity.

We have found that RNA and DNA probes of fur and furin-specific antisera can be used as a diagnostic aid for the detection of tumors, in particular non-small-cell lung carcinomas, breast tumors (mamma carcinomas), intestinal tumors (in particular colon carcinomas), urogenital tumors (in particular prostatic carcinomas) and hematological malignities, as fur is not expressed in some normal tissues, such as normal lung tissue of man, rat and cat, but pronounced expression occurs in certain tumors, in particular non-small-cell lung tumors, breast tumors (mamma carcinomas), intestinal tumors (in particular colon carcinomas), urogenital tumors (in particular prostatic carcinomas) and hematological malignities.

The detection of tumors, such as in particular non-small-cell lung carcinomas, breast tumors (mamma carcinomas), intestinal tumors (in particular colon carcinomas), urogenital tumors (in particular prostatic carcinomas) and hematological malignities can be based on hybridization experiments, in which RNA or DNA probes of fur are used as a diagnostic aid to determine the possible presence of fur mRNA. Hybridization experiments have shown the diagnostic utility of fur probes.

For the detection of tumors, such as in particular non-small-cell lung carcinomas, breast tumors (mamma carcinomas), intestinal tumors (in particular colon carcinomas), urogenital tumors (in particular prostatic carcinomas) and hematological malignities, antisera having a furin specificity can also be used. For such a purpose, various techniques are known, such as routine immunofluorescence, ELISA, Western blotting (Methods in Enzymology, Vol. 104, Acad. Press. Inc. (1984)), or immunoprecipitation combined with polyacrylamide gel electrophoresis (Van de Ven et al., Virology 8, 334-344 (1977)).

For detection, human and animal tissues or body fluids, such as sputum, urine, blood, and the like can be used. The use of sputum and of tissue material obtained by biopsy has been found to be very suitable.

The invention will now be elucidated in and by the following detailed description and experimental section.

The genome of a number of independent feline and avian sarcoma virus isolates contains transduced c-fes/fps proto-oncogene sequences of cat and chicken. Mobilisation of their transforming potential turned out in all cases investigated to be combined with a fusion of c-fes/fps proto-oncogene sequences to retroviral gag gene sequences. Although the function of the c-fes/fps proto-oncogene is not yet known, it could be of particular importance in hematopoietic multiplication, because expression appears to be substantially restricted to hematopoietic tissue. Expression of the c-fes/fps proto-oncogene has been found in normal and leucemic myeloid cells and cell lines of a number of species. A tyrosin-specific protein kinase with a molecular weight of about 92,000 has been described as its most important translational product. For the c-fes/fps mRNA lengths of 2.6 to 3.2 kb have been described in the literature. The genetic organization of the fes/fps proto-oncogene, however, has not as yet been completely determined. The isolation and genetic organization of c-fes/ fps proto-oncogene sequences in man and cat, and their malignant potential upon fusion thereof with gag gene sequences of feline leukaemia virus origin have been described by Roebroek et al., EMBO Journal 4 (1985), 2897-2904 and Verbeek et al., Gene 35 (1985), 33-34. In the former article, the topographical distribution of nearly all v-fes /fps homologous sequences in man has been derived from the complete nucleotide sequence of a human 13 kb EcoRI/EcoRI restriction fragment. A sequence homologous to 12 base pairs at the 5′ terminal of the v-fes viral oncogene of the Gardner-Arnstein strain of feline sarcoma virus (GA-FeSV), described by Hampe et al., Cell 30 (1982), 775-785, and 140 base pairs at the 5′ terminal of v-fps of Fujinami sarcoma virus (FSV), described by Huang et al., J. Mol. Biol. 181 (1985), 175-186, were still absent. In the experimental section of this description, the position of the missing 12 bp in the human and feline proto-oncogene are identified. Also, further data are presented about the 5′ and 3′ terminal of the human c-fes/fps transcription unit and about the newly discovered transcription unit in the area directly upstream of the v-fes/fps homologue sequences, which new transcription unit is referred to herein as fur . The knowledge concerned was collected by comparing nucleotide sequence data of the 5′ region of a fes/fps viral oncogene with those of the v-fes/fps homologue regions of man and cat, in which the position of the 3′ portion of an as yet unidentified c-fes/fpsexon was found. By comparative Southern blot and heteroduplex analysis of human and feline DNA immediately upstream of the v-fes/fps homologue regions, an extensive but discontinuous homology over a length of 9 kb was found, which DNA section belongs to the fur gene. Northern blot analysis of mRNA from KG-1 myeloid cells with fes /fps or fur-specific probes resulted in the discovery of a 3.0 kb fes/fps and a 4.5 kb fur transcript. Analysis of a number of tissues of an addult Wistar Lewis rat for the presence of fur transcripts disclosed a differentiated expression pattern thereof. From an oligo(dT)-primed KG-1 cDNA collection, a 0.95 kb cDNA recombinant clone with fes/fps information and a 2.2 kb cDNA recombinant clone with fur information were isolated. Comparative nucleotide sequence analysis of the fes/fps cDNA and the corresponding region in the human genome showed that the cDNA contained genetic sequences identical to and colinear with exons 15-19 and that the poly(A) addition signal immediately downstream of exon 19 was functional. A similar analysis of the 2.2 kb fur cDNA proved that the poly(A) addition signal of the fur transcript is close to the newly discovered fes/fps exon. The intermediate region contained a CATT sequence but no 'TATA' box. The fur transcript was found to have a long non-coding section at its 3′ end with a length of about 1600 bp. The genetic sequences for fur and for c-fes /fps turned out to be spaced apart less than 1 kb in man and in cat. The transcription direction of fur and of fes/fps turned out to be equal.

The discovered conserved proximity of fur and fes/ fps is remarkable and could have implications for the expression of these genetic sequences or constitute an indication for a functional relationship between their translational products. The expression of the c-fes/fps proto-oncogene is substantially restricted to cells of hematopoietic origin. The above protein with a molecular weight of about 92,000 (in mammals; in birds the protein has a molecular weight of about 98,000), which is the translational product of the c-fes/fps proto-oncogene, has a tyrosine-specific protein kinase activity which is attributed to a protein domain in the carboxy-terminal portion of the protein. The precise function of the proto-oncogene product is not as yet known, however. Possibly, the fes/fps protein forms a receptor, as is the case with some other proto-oncogenes encoding proteins with a tyrosine-specific protein kinase activity and receptor characteristics. These include, for example, the c-fms proto-oncogene, which is homologous to, or identical to, the gene coding the receptor for the mononuclear phagocyte growth factor CSF 1 or the c-erb B proto-oncogene, which is assumed to encode the epidermal growth factor receptor. Receptors generally appear to contain protein domains which are also found in certain other proteins. A mechanism which could explain the formation of receptors is exon shuffling. In the case of the c-fes/fps protein, nucleotide sequence data of the human gene do indicate a protein kinase domain, but no other receptor characteristics. The characterization of fur and the translational product thereof could be of interest to gain a better insight into this. For this purpose, and as will follow from the experimental section of this description, the fur gene and the gene product designated furin has been investigated. By determining the nucleotide sequence of a 3.1 kb fur specific cDNA isolated from a human cDNA collection, an open reading frame of 1498 bp was found, on the basis of which the 499 carboxy-terminal amino acids of the primary translational product of fur could be determined. By computer analysis we determined that furin contains a possible transmembrane domain similar to that of class II MHC antigenes. Furthermore, a cystein-rich region was found to be present. This exhibited a significant degree of homology, in particular with regard to the topography of the cystein groups with the cystein-rich regions of the human insulin receptor and the human epidermal growth factor receptor. Accordingly, the furin appears to have receptor-like characteristics.

The fur transcription unit appears to be distributed over a DNA area with a length of about 10 kb. The amino acid sequence derived from the open reading frame of about 1500 nucleotides contains a hydrophobic region as found in transmembrane domains, which hydrophobic region is in a position approximately 50 amino acid residues upstream of the carboxy terminus. Upstream of the transmembrane domain, there is a cystein-rich region. As stated before, the transmembrane domain greatly resembles the transmembrane domains found in proteins coded for by class II genes of the major histocompatibility complex (MHC). Cystein-rich regions are characteristic of various receptor proteins and are to be found in human insulin receptor (HIR), the human epidermal growth factor receptor (HER), the glucocorticoid receptor, the oestrogen receptor and the low-density lipoprotein receptor. Significant homology, especially with regard to the topography of the cystein groups was found between the cystein-rich regions of HER, HIR and furin. As the presence of a transmembrane domain adjacent to a cystein-rich region, as is the case in the translational product of fur, is more often observed in receptors, there are strong indications that furin is a receptor for an as yet unknown ligand.

On account of the structural properties of furin, an investigation into the expression pattern of fur in normal and malignant tissues is interesting. In that way, indications could possibly be obtained with regard to the receptor type and nature of its ligand. The fact that the fur gene is located in close proximity to the fes/fps proto-oncogene could have implications for the expression of these genetic sequences. As explained in the experimental section of this description, we have therefore investigated the expression of the fur gene by means of Northern blot analysis. In normal tissue of various histological types, the fur gene was found to exhibit a differentiated expression pattern. In samples of normal lung tissue, the expression level was very low. In the investigation, we also analyzed biopsy material of various types of human lung carcinomas for fur expression. In the investigation, lung samples of 7 patients with small-cell lung carcinomas (SCLC) and of 30 patients with non-small-cell lung carcinomas (NSCLC) were included. Of the six SCLC cases, five exhibited a fur expression level comparable to that of normal lung tissue. In the one remaining case, only a slightly elevated expression level was observed. Very high quantities of fur transcripts, however, were found in the lung tumor samples of all patients with NSCLC, except in the tumor of one patient, in which a relatively low level was detected. Southern blot analysis did not show any gene amplification of fur in NSCLC. The results do show that the fur gene is a suitable tumor marker for distinguishing between SCLC and NSCLC.

## Experimental section

## MATERIALS AND METHODS

### Cell line and cosmid clones

The permanent KG-1 human cell line obtained from bone marrow cells of a patient with erythroleukaemia that developed into accute myelogenous leukaemia has been described by Koeffler et al., Science 200 (1978), 1153-1154. Isolation of the human cellular homologue of v-fes/ fps was effected according to Groffen et al., Science 216 (1982), 1136-1138. In the above article by Roebroek et al., subclones in pSP64 of a 13 kb Eco RI/EcoRI DNA fragment containing all v-fes/fps homologous sequences, and of a 9.2 kb HpaI/ EcoRI DNA fragment flanking the former fragment at its 5′ end are described. A 20 kb EcoRI/EcoRI fragment flanking the former 13 kb DNA fragment at the 5′ end was subcloned in pUC18. The feline v-fes/fps cellular homologue was isolated from a feline cosmid collection (see the above article by Verbeek et al.).

### DNA probes and hybridization

Probes were isolated and labeled by the method of Van den Ouweland et al., Biochim. Biophys. Acta 825 (1985), 140-147. Hybridization experiments on nitrocellulose membranes were carried out in the manner described in it, and for hybridizations on nylon membranes (Hybond N, Amersham) the method of Church and Gilbert, Proc. Natl. Acad. Sci. USA 81 (1984), 1991-1995 was used. The nylon membranes were dehybridized by incubation in 5 mM Tris-HCl (pH 8.0), 2 mM EDTA and 0.1 x Denhardt's solution (1x Denhardt's solution contains 0.02 % (w/v) bovine serum albumin, 0.02 % (w/v) polyvinyl pyrrolidone and 0.02 % (w/v) ficoll), the hybridization being carried out at 65°C for 2 hours. If necessary, the procedure was repeated up to three times. The dehybridization was always checked by autoradiography. Blots could be used several times, mostly up to six times without significant loss of signal.. To investigate the fur expression, we used a 3.1 kb fur-specific cDNA probe isolated from an oligo(dT) primed cDNA library of KG-1 cells. We also used a 0.95 kb fes/fps-specific cDNA probe comprising the genetic sequences of the last five exons of c-fes/fps . In the investigation for the presence of actine transcripts, we used pAct-1, a hybrid molecule of pBR322 and 1.2 kb actine-specific cDNA sequences of hamster origin.

### Heteroduplex analysis

Electron microscopic analysis of duplexes between the 9.2 kb human HpaI/EcoRI DNA fragment and the 14 kb feline EcoRI/EcoRI DNA fragment was performed by the method of Davis et al., Methods Enzymol. 21 (1979), 413-428. Grids were examined in a Zeiss EM109 electron microscope at 40 kV. Electron micrographs were taken at a magnification of 6,000 x. Measurements were made on 25 heteroduplex structures.

### mRNA isolation and Northern blot analysis

Total cellular RNA was isolated by means of the lithium-urea procedure described by Auffray and Rougeon, Eur. J. Biochem. 107 (1980) 303-314. Ten µg oligo(dT) cellulose purified mRNA was glyoxalated and size fractionated on 1.0 % agarose gels and transferred to Hybond-N (procedure as recommended by Amersham).

## Construction and screening of cDNA library

By means of poly(A)-selected RNA from KG-1 cells, a cDNA library was constructed in λgt11 by the method of Huynh et al., DNA cloning Techniques, a Practical Approach (1985), published by Glover, IRL Press, Oxford. As primers, we used oligo(dT) or a mixture of oligo(dT) and random-synthesized hexanucleotides. The cDNA reaction was modified according to Gubler and Hoffmann, Gene 25 (1983), 263-269. The first strand reaction was modified by denaturing the template RNA prior to the first strand synthesis by means of methyl mercury hydroxide. By means of this procedure, the average length of the first strand reaction products was increased by about 30 %. About 450,000 plaques obtained upon infection of E. coli Y1090, described in the above work by Huynh, were screened as described by Hanahan and Meselson, Gene 10 (1980), 63-67.

## DNA sequence analysis

DNA fragments were inserted into the polylinker region of M13mp8-11 (Messing en Vieira, Gene 19 (1982), 269-276). All DNA sequences were determined by the dideoxy-sequencing method of Sanger et al., Proc. Natl. Acad. Sci. USA 74 (1977), 5463-5467. All parts of the reported DNA sequence were obtained from both strands of the cloned DNA. The gel readings were recorded, edited, and compared using the Staden programmes (Nucl. Acids Res. 10 (1982), 2951-2961).

## Computer analysis

Protein sequence homology was studied with the protein sequence database (release 5.0) of the Protein Identification Resource (PIR) of the National Biomedical Research Foundation and with PGtrans protein database (release 35.0) of the Pasteur Institute at Paris. The DNA sequence analysis was performed with the EMBL nucleotide sequence database (release 7). The computer analysis was performed on a VAX 11/780 computer with programmes according to Lipman and Pearson, Science 227 (1985), 1435-1441).

## Lung carcinomas

Biopsy specimens of lung carcinomas were removed from 37 lung cancer patients by surgery. The specimens were frozen immediately after removal and were preserved in liquid nitrogen. Tumors were characterized by routine microscopical and histopathological techniques and electron microscopy, and classified according to the criteria of the World Health Organization as small-cell lung carcinomas (SCLC, 7 cases) or non-small-cell lung carcinomas (NSCLC, 30 cases). Of the NSCLC cases, 14 were squamous cell lung carcinomas and 16 adenocarcinomas.

## INVESTIGATION AND RESULTS

### Comparison of human and feline DNA immediately upstream of the v-fes/fps homologous regions

The topography of most of the human v-fes/fps homologous genetic sequences have been described before. Nucleotide sequence analysis of a 13 kb EcoRI/EcoRI DNA fragment showed they were split up into at least 19 exon segments, and of these exons 2-19 were characterized before; see the above article by Roebroek et al.. As stated before, genetic sequences at the 5′ end of v-fps of FSV or v-fes of GA-FeSV remained unaccounted for. In KG-1 cells, which show a high level of expression of the fes/fps proto-oncogene, no such transcripts could be detected in a Northern blot analysis with the 1 kb DNA region upstream of exon 2 as a probe. In an attempt to identify the missing sequences in the human genome and to investigate whether the region immediately upstream of the v-fes/fps homologous sequences contained additional sequences that were not represented in any of the known v-fes/ fps isolates, but did belong to the c-fes/fps proto-oncogene, human DNA upstream of the v-fes/ fps homologous region (a 9.2 kb HpaI/Eco RI DNA fragment) was compared with a 14 kb EcoRI/EcoRI DNA fragment representing the corresponding feline region, by Southern blot analysis. The 9.2 kb HpaI/EcoRI human DNA fragment shown in Fig. 1A was digested with restriction endonucleases EcoRI, KpnI, PstI, Xba I, and XhoI or various combinations of these restriction endonucleases in double digestions, and hybridizations were performed under conditions of high stringency with the 14 kb EcoRI/Eco RI feline DNA fragment, also shown in Fig. 1A, as a molecular probe. It was found that there was an extensive and strong homology between the two fragments. In a reciprocal experiment, similar restrict endonuclease digestions of the feline 14 kb EcoRI/Eco RI DNA fragment and hybridization analysis with the human 9.2 kb HapaI/EcoRI DNA fragment as a probe exhibited the same results. As shown diagrammatically in Fig. 1A, homology between the two fragments starts almost immediately upstream of the v-fes/fps homologous regions and extends over a DNA region of about 9 kb. This fes/fps upstream region was designated fur.

To further characterize the furregion in man and cat, heteroduplex analysis was performed with the human 9.2 kb HpaI/EcoRI and the feline 14 kb Eco RI/EcoRI DNA fragments. The results of this heteroduplex analysis are shown in Fig. 1B. The experiment confirmed the observations of the Southern blot experiments and further showed that there are at least 4 non-homologous regions among the homologous genetic sequences. In a number of the 25 heteroduplex structures that were analyzed, free regions of apparently reduced homology were observed. Their positions and frequencies are indicated by arrows in Fig. 1C.

## Transcription of the human DNA upstream of the v-fes/fps homologous region

To establish whether the DNA directly upstream of the v-fes/fpshomologous region was a part of the proto-oncogene, we studied c-fes /fps transcription in the human KG-1 cell line. RNA was isolated from these cells, poly(A) selected, size fractionated on a 1 % agarose gel and blotted onto Hybond N. Expression of c-fes/fps proto-oncogene sequences could be readily detected with the $S_r$ v-fes-specific probe of Franchini et al., Nature 290 (1981), 154-157. A transcript with a length of 3.0 kb was found. Hybridization of the same Northern blot with a number of probes each representing a different part of the 9.2 kb Hpa I/Ecori human fur region revealed a 4.5 kb transcript. The probes used are shown in Fig. 2. The autoradiogramm (not shown) showed in the case of probe 1 a 4.5 kb transcript and a descending smear of radioactivity; this could be due to the presence of recurrent sequences in the probe used (a 2.35 kb HpaI/BamHI DNA fragment). From these Northern blot experiments we concluded that human fur (and also feline fur, data not shown) constituted a transcription unit for a 4.5 kb mRNA different from the c-fes/ fps transcript detected with the $S_r$ probe. The fact that probe 3 also strongly hybridized with the 4.5 kb fur transcript indicated that the fur sequences found are located in close proximity to the 5′ v-fes/fps homologous genetic sequences, as is also apparent from Fig. 1A.

Analysis of fur expression was also studied in a number of tissues of an addult Wistar Lewis rat. The tissues studied included brain, heart muscle, kidneys, lung, testis and thymus. High levels of the 4.5 kb furtranscript were found in brain, kidney ~~and~~ thymus. Very low levels of fur transcripts appeared to be present in heart muscle, lung and testis. These data indicate a differential expression pattern of the fur transcription unit. To get more insight into the genetic organization of the fur and fes /fps transcription units, nucleotide sequence data of the transcripts and their genomic counterparts should be compared.

## Molecular cloning and DNA sequence analysis of human c-fes/fps-related and fur-related cDNA

To isolate c-fes/fps-related and fur-related cDNA, an oligo(dT) primed cDNA library in λgt11 was constructed using KG-1 mRNA as a template. In screening 4.5 x 105 plaques with the v-fes-specific $S_r$ probe, a cDNA clone was isolated which contained a c-fes/fps-specific insert of 0.95 kb. The same library was also screened with a human fur specific probe, namely, probe 3 of Fig. 2. This resulted in the isolation of a cDNA clone with a 2.2 kb fur-specific insert. Both cDNA inserts were subcloned in M13mp10 for DNA sequence analysis.

As stated before, the topography of the exon sequences in the human c-fes/fps proto-oncogene has been determined earlier on the basis of homology with v-fes/fps. The 3′ border was tentatively placed at a position approximately 200 bp downstream of a stop codon in exon 19 where a possible polyadenylation signal is present. The availability of nucleotide sequence data of the 0.95 kb human fes/fps-related cDNA (Fig. 3) and previously published sequence data of genomic fes/fps DNA (Roebroek et al.) made possible a comparative analysis. The 5′ end of the cDNA clone was found to start 5 nucleotides downstream of the 5′ end of exon 15 and at the 3′ end the cDNA clone contained a poly(A) stretch representing the poly(A) tail. Therefore, the 3′ border of the fes /fps transcript in KG-1 cells could be determined rather precisely. The data confirmed the previous suggestion by Roebroek et al. that the poly(A) addition signal at a position about 200 bp downstream of the stop codon in exon 19 is functional. It could further be established that the poly(A) addition site was 17, 18 or 19 bp downstream of the polyadenylation signal. No differences were found between the nucleotide sequence of the human c-fes/fps-related cDNA here described and the corresponding genomic region of the human fes/fps proto-oncogene. The exon/intron arrangement of the exons 15-19 as proposed by Roebroek et al. also proved to be correct.

Although the c-fes /fps-related cDNA only represented one-third of the observed mRNA length, some additional characteristics of the c-fes /fps transcription unit could be derived from analysis of the fur transcription unit. A comparative analysis of nucleotide sequence data of the 2.2 kb fur-related cDNA and its corresponding and flanking region in genomic DNA revealed that the nucleotide sequence of the cDNA insert was identical to that of the genomic DNA fragment (Fig. 4). These sequences are indicated by capital letters in Fig. 4. At its 3′ end, the fur cDNA had a poly(A) stretch marking the end of the fur transcription unit. At precisely 21 bp upstream of the poly(A) addition site, there was an ATTAAA consensus sequence of a poly(A) addition signal (Wickens & Stephenson, Science 226 (1984), 1045-1051). Comparative sequence analysis further indicated that the 2.2 kb fur cDNA was contained in a single exon, referred to herein as Z. To obtain information about a possible fur translational product, we compared the three reading frames in exon Z. In all three reading frames, various stop codons were present The first stop codon was found in each of the three different reading frames in the first 420 base pairs from the 5′ end of the cDNA clone. From this it was concluded that the fur mRNA has a long non-coding region at its 3′ end.

The 3′ end of the fur transcription unit was found to be in close proximity to the v-fes/fps homologous region in man and cat. As stated before, in front of exon 2 of c-fes /fps 12 base pairs at the 5′ end of v-fes of GA-FeSV and 140 base pairs at the 5′ end of v-fpsof FSV had to be accounted for. In the case of the 140 base pairs of v-fps, it may well be that, in human c-fes/fps no sequences homologous to these 140 base pairs could be identified because of the divergence of non-coding exon sequences. In the case of the 12 base pairs of v-fes, it could not be excluded that homologous sequences were not recognized because comparison was limited to a DNA stretch of only 12 nucleotides. In order to obtain more clarity, we sequenced the feline c-fes/fps proto-oncogene region that contained exon 2 and about 1 kb of upstream flanking sequences. The sequence data, not shown herein, supported the boundary assignment of human exon 2 as suggested by Roebroek et al.. The homology between the genetic sequences in exon 2 of man and cat was about 85 %. Upstream of exon 2, homology was about 65 %. In this region in the feline DNA, a stretch of 12 base pairs was found, which was identical to the 12 base pairs at the 5′ end of the feline v-fes oncogene. In the human DNA, a virtually identical stretch was found at a similar position. Of these bases, 11 were identical to those of the viral oncogene and of the feline proto-oncogene. An insertion of 1 nucleotide was observed in the human DNA stretch. The 3′ end of both the feline DNA stretch and the human DNA stretch (see Fig. 4) was followed by a sequence characteristic for a splice donor site (Mount, Nucl. Acids Res. 10, (1982), 459-472). These data suggest that these stretches overlap the 3′ region of a new human and feline c-fes/fps exon. The 3′ boundary of exon 1 and the 5′ end of exon 2 in human and feline DNA are compared in Fig. 4. The 5′ boundary of exon 1 remains to be established, but, based upon the size of the polyadenylated mRNA, detected in KG-1 cells, the size of the first c-fes/fps exon can roughly be estimated at 200 nucleotides.

The location of the 3′ end of the fur transcription unit in relatively close proximity to the fes/ fps exon 1 (see Fig. 5) raises the question whether the promotor of the fes/fps transcription unit is located between the fur exon Z and the fes/fps exon 1. A search for sequences typical of promotor regions revealed a sequence that is in good agreement with a CATT box, namely the GGCCATTCT sequence at position 3093-3101 in Fig. 4. The sequence data available for feline c-fes/fps indicate that the GGCCATTCT sequence is also present at a similar position in the feline DNA. However, no sequences resembling the consensus sequence of a TATA box could be identified. The potential CATT box would be in an appropriate position relative to the 5′ end of the transcription unit to accommodate a c-fes/ fps transcript of 3.0 kb as found in KG-1 cells. However, more data are required to locate the 5′ end of the proto-oncogene and to identify the DNA sequences that control its expression. The observation that, in man and cat, the linkage of the fur and fes/ fps transcription units has been conserved during evolution is interesting, because it could have functional implications and can help in elucidating the regulation of the expression of c-fes/fp s.

Topography of the fur transcription unit

To investigate whether human sequences located further upstream would hybridize with topographically corresponding feline sequences, we also used an EcoRI/EcoRI DNA fragment with a length of about 20 kb. This fragment was located right next to the 5′ end of the 13 kb EcoRI/EcoRI DNA fragment containing the human v-fes/fps cellular homologue (see Fig. 6). Comparative Southern blot analysis with the insert of feline cosmid clone cos-1 (see the above article by Verbeek et al.) showed that the homology between men and cat in the region located upstream of fes/fps extended about 1 kb further upstream beyond the HpaI restriction endonuclease cleavage site (Fig. 6). The human and feline genetic sequences were found to diverge upstream of the PstI cleavage site, which gave an indication of the position of the 5′ boundary of the fur transcription unit. A support for this is given by the finding that within the 10 kb fur region, only the 2.4 kb HpaI/BamHI DNA fragment (Fig. 6) showed promotor activity in a chloramphenicol acetyl transferase assay (70 % of the activity of the promotor in the LTR of Rous sarcoma virus).

In connection with a further characterization of the genetic organization of fur and the transcription unit in this region, a cDNA library was constructed in λgt11 by means of poly(A)-selected RNA from KG-1 cells. Using the 0.7 kb Xho I/XhoI DNA fragment as a molecular probe (Fig. 6), a 3.1 kb fur-specific cDNA was isolated. The nucleotide sequence of the 3.1 kb cDNA clone was determined, and from this it turned out that it was 3080 base pairs long (without the poly(A) tail portion) and included the poly(A)-addition signal and the poly(A)-addition site (Fig. 7). When the 3.1 kb cDNA clone was used as a molecular probe in a Northern blot analysis, the previously described 4.5 kb fur mRNA was detected as the only transcript.

To determine the genomic organization of the sequences corresponding to the molecularly cloned fur-specific cDNA, we also determined the nucleotide sequence of the genomic DNA regions hybridizing with the 3.1 kb cDNA probe (Fig. 7). A comparison of the cDNA and the genomic nucleotide sequence indicated that the 3.1 kb transcribed sequences were distributed over 8 exons within a 5.4 kb genomic DNA region (for a diagrammatic representation reference is again made to Fig. 6). These exons are designated herein by letters S to Z. In the nucleotide sequence of the 3.1 kb cDNA clone, we looked for the presence of a large open reading frame. The above study of a 2.2 kb fur-specific cDNA clone revealed various stop codons in all reading frames. In the 3.1 kb cDNA clone, however, an open reading frame of 1498 base pairs was found which started at the 5′ end of the clone (Fig. 8). The identification of the open reading frame in the transcribed genetic sequences in the fur region opens up the possibility to learn more about the possible function of its translational product.

Computer analysis of the putative fur translational product

From the 1498 bp open reading frame, the amino acid sequence was deduced (Fig. 9A). For the matter of that, this sequence only represents a carboxy terminal portion of the putative fur translational product, whose complete size and amino acid sequence remains still to be established. As stated before, this fur translational product is referred to herein as furin. The carboxy-terminal portion of furin has also been analyzed using a hydrophobicity plot (Kyte and Doolittle, J. Mol. Biol. 157 (1982), 105-132), see Fig. 9B. This analysis indicated a stretch containing many hydrophobic amino acids (the amino acid residues 418-450) indicating that it might function as a transmembrane domain. This putative transmembrane domain was located about 50 amino acid residues from the carboxy terminus of furin. Screening of the PIR and PGtrans databases showed that this domain was highly homologous to the transmembrane domain of class II MHC antigens (Schenning et al., EMBO J. 3 (1984), 447-452). Homology was 41 % in the stretch from 422 to 445, not including conservative amino acid substitutions. When these were taken into account, the homology would be 64 % (see Fig. 9C).

A further characteristic feature of furin is the presence of a cystein-rich region (spanning from amino acid residue 277 to 411). Recent studies indicate that cystein-rich regions are characteristic for certain types of receptors. Screening of the PIR and PGtrans databases with the deduced furin sequence revealed a strong homology between its cystein-rich region and that of the human insulin receptor (Ullrich et al., Nature, 313 (1985), 756-761) and the human epidermal growth factor receptor (Ullrich et al., Nature 309 (1984), 418-425); see Fig. 10. The homology turned out to extend over a stretch of 135 amino acid residues. Overall homology between furin and human insulin receptor was, in this stretch, 21 %. The cystein topography in the cystein-rich regions of the three proteins, however, exhibited great similarities. Between furin and the human insulin receptor, the position of 9 out of 12 cystein residues was found to be the same. In Fig. 10, the cystein-rich regions of these three proteins are shown, with the cystein residues being shown in boxes.

In view of the cystein-rich region and the transmembrane domain in the putative translational product of fur, the furin appears to be a protein having receptor-like features. A consensus is emerging that receptors, being multifunctional proteins, contain multiple domains each with its own function. A good example is the human low-density lipoprotein (LDL) receptor which contains one domain that is homologous to the precursor of the epidermal growth factor, one domain that contains sequences homologous to complement component C9 and is located in a cystein-rich region, one domain where O-linked sugars are clustered, and a transmembrane domain. Other characterized receptors are the human insulin receptor, which shows some homology to the c-ros proto-oncogene, the human epidermal growth factor receptor which is related to the c-erb B protooncogene, the human oestrogen receptor and the glucocorticoid receptor, which are both related to the v-erb-A proto-oncogene, and the c-fms /CSF-1 receptor. Some of these receptors are characterized by a cystein-rich region, which is probably involved in ligand binding, so that they appear to belong to the same family of receptors. Other receptors lacking the cystein-rich region may belong to a different family and are likely to control transmembrane signal generation in an alternative way. The results of the computer analysis seem to indicate that furin belongs to the same family of receptors as the human insulin receptor and the human epidermal growth factor receptor, and acts in a similar way. As regards the position of the transmembrane domain, furin resembles the LDL receptor in that its transmembrane domain is located at the carboxy-terminal portion of the protein. Only 50 amino acid residues seem to be located on the cytoplasmic side of the plasma membrane and to project into the cytoplasm. An interesting fact is that the LDL receptor belongs to a class of receptors that carry their ligands into cells after clustering in clathrin-coated pits.

An interesting aspect of fur is its conserved close proximity to the fes/fps proto-oncogene sequences. It is not as yet clear whether fur and fes /fps belong to the same locus or represent independent genes. Nucleotide sequence data on the DNA region downstream of the 3′ non-coding exon of fur (see Fig. 6) and the 5′ fes /fps exon sequences failed to provide sufficient evidence to identify a promotor in this region. It cannot be excluded, therefore, that the promotor region for the fes/fps sequences is located somewhere further upstream. Possibly, the translational products of fur and fes/fps are functionally implicated and together constitute a receptor similar in its way of action to the human insulin receptor and the human epidermal growth factor receptor. In such a model, the c-fes/fps tyrosine-specific protein kinase domain would be linked to the fur ligand binding domain. In the human epidermal growth factor receptor, these domains are present in a single polypeptide and in the human insulin receptor in two polypeptides derived from a single precursor molecule. Since fur exhibits a differential expression pattern in normal tissue and fes/fps expression appears to be restricted to hematological cells of the moncyte/macrophage and granulocyte lineage, this model seems only valid in these particular cells. The mosaic nature of the structural organization of receptors with domains shared by different proteins, however, could imply that they are multifunctional. One example is the epidermal growth factor precursor, which may be a receptor for an unknown ligand in addition to being the precursor for the epidermal growth factor. Since truncated forms of the fes/ fps protein possess transforming capabilities, elucidation of the function of fur may also shed light on the mechanism of cell transformation by c-fes/fps-derived oncogenes.

Differential expression of fur in normal tissues

The expression pattern of the fur gene in normal tissue of different histological types was studied by means of Northern blot analysis, using a suitable fur-specific probe. When a combination of the genomic probes 1, 2 and 3 is used, the 4.5 kb fur transcript could be detected in poly(A)-selected RNA of KG-1 cells. However, a smear of transcripts of lower molecular weight was always observed. When a 3.1 kb fur-specific cDNA clone was used, however, a clear and distinct band was obtained instead. This cDNA clone represented about 70% of the 4.5 kb fur-transcript and contained the genetic sequences that encode the transmembrane domain and the cystein-rich region. The smearing effect is probably due to recurrent sequences most likely located in the 5′ region of fur. For a further study of the fes/ fps transcripts, a 0.95 kb fes/fps-specific cDNA was used instead of the v-fes-specific $S_L$ probe.

Poly(A)-selected RNA from rat tissue samples was size fractionated in agarose gels and transferred to Hybond paper for hybridization analysis. High levels of fur transcripts were found in RNA from tissue samples of brain, kidneys, spleen, thymus and particularly liver, while only low levels were detected in heart muscle, lung and testis. Similar results were obtained with RNA from various tissues of African green monkeys. It is not as yet clear whether the weak hybridization signals are due to low levels of fur transcripts in the tissue-specific cells of these specimens or to the presence of cells of hematopoietic origin, which exhibit relatively higher levels of fur expression. At any rate, the results indicate that the fur gene is differentially expressed in a tissue-specific manner. In situ hybridization of immunofluorescence analysis could establish in more detail which cell types contain the fur transcripts or the translational product thereof.

When the same blots were assayed for the presence of fes/fps-related sequences, a hybridization signal was observed only in spleen samples. This observation is in agreement with earlier findings indicating that expression of fes /fps is mainly restricted to hematopoietic cells of the myeloid lineage.

Expression of fur in human lung carcinomas

In connection with the observation that the level of fur transcripts is very low or absent in heart muscle, lung and testis, we also studied the fur expression in naturally occurring tumors. In man lung cancer is the most important cause of cancer death in the Western world and lung cancer incidence has dramatically increased over the last 50 years. Partly in view of this, the study was directed in the first instance to lung carcinomas.

Included in the study were 7 cases of SCLC and 30 cases of NSCLC. The latter 30 cases included 14 cases of squamous cell lung carcinoma and 16 cases of adenocarcinoma. For control experiments, human lung tissue from healthy individuals was used, and also lung tissue from normal cat, mouse and monkey. A Northern blot analysis was performed in which equal amounts of poly(A)-selected RNA of different types of lung carcinomas and of different control lung tissues were used. To ensure that the samples tested contained equal amounts of RNA, the $OD_{260}$, ethidiumbromide staining, and actin transcripts levels were measured. In all experiments, experimental conditions were the same. Normal lung tissue of man, cat, mouse and monkey exhibited very low levels of fur expression. Similarly, in SCLS, fur expression was also just above detection level. In the NSCLC, however, high levels of fur transcripts were observed. No differences were found in the levels of fur expression in squamous lung cell carcinomas and adenocarcinomas. The levels of fur transcripts in NSCLC are estimated to be at least 30 times as high as in normal lung tissue or in SCLC. The results are summarized in Table A. In the sample of one of the seven SCLC patients the fur levels were elevated to some extent. The six others were all very low. In one of the 14 adenocarcinoma patients, the level of fur transcripts was low. Both cases could be explained from the fact that lung carcinomas sometimes exhibit a phenotypic heterogeneity.

The same Northern blots were also used to study the expression of the fes/fps proto-oncogene. Its expression appeared to be low in all lung tissues tested, although some variation seemed to exist in the lung tumors. The observation that the fes/fps expression in lung tissue is low is in agreement with earlier reports that indicated a restriction to hematopoietic cells. The fact that in NSCLC, the expression of the fur gene is highly elevated, but the fes/fps proto-oncogene in its immediately downstream region is not, is a clear indication that different regulatory systems exist for each of these two transcription units. A cell type with high levels of fes/fps expression and low levels of fur expression has not as yet been found.

The approximately 30 times higher expression of fur transcripts in lung tumor samples from patients with primary adenocarcinomas or squamous lung cell carcinomas as compared to normal lung tissue or SCLC is a property by virtue of which the fur gene can be used as a marker in studies on human lung cancer or as a diagnostic aid in the detection of tumors, such as in particular non-small-cell lung carcinomas. A number of biomarkers are known which are being used in lung cancer research. These include L-3,4-dihydroxy-phenylalanine decarboxylase, bombesin and bombesin receptor, epidermal growth factor receptor, peptide hormones such as calcitonin, ACTH, AVP, neurotensin, neuron-specific enolase, creatine kinase (BB isoenzyme) and intermediate filament proteins. Most of these markers can be used to detect differentiated functions associated with SCLC or to discriminate between classic and variant SCLC. Only the epidermal growth factor receptor is useful as a marker for non-small-cell lung carcinomas, which constitute approximately 75-80 % of all new cases of primary lung cancer. The availability of fur as a new marker for NSCLS opens up the possibility of early lung cancer diagnosis. The fact that, in such tumors, the level of fur transcripts is highly elevated is an effect by virtue of which a sensitive test can be developed.

The fur gene is not amplified in NSCLC

The high levels of fur transcripts in NSCLC could be explained from gene amplification in these tumors. It is known that myc cellular oncogenes are amplified in lung tumors. To test the possibility of fur amplification in human NSCLC, we assayed equal amounts of genomic DNA from normal lung tissue of different individuals, and also from SCLC and NSCLC for the presence of fur-specific genetic sequences. For Southern blot analysis, genomic DNA was incubated with restriction-endonuclease Pst I. As molecular probes, the 3.1 kb fur cDNA clone or a combination of probes 2 and 3 was used. The results of these experiments indicated that in none of the DNA samples tested amplification of the fur gene had occurred. Furthermore, no major rearrangements in the fur or the fes/fps (tested with the complete viral oncogene of the Gardner-Arnstein strain of feline sarcoma virus as a probe) regions could be observed. These results clearly demonstrated that fur amplification in NSCLC is not due to fur amplification.

Expression of fur cDNA in bacteria

The 3.1 kb fur cDNA (EcoRI/EcoRI DNA fragment) was digested with the restriction-endonuclease SmaI, and the resulting SmaI-SmaI fur DNA fragment was purified by means of agarose gel electrophoresis. Subsequently, HindIII linkers were attached to the purified fragment by means of DNA ligase. After treatment of the resulting DNA with the restriction-endonuclease HindIII, the cleaved DNA fragment was cloned in the HindIII site of pUR292. This resulted in pMJ109.

The fur DNA insert of pMJ109 was cleaved from the vector by means of the restriction-endonuclease HindIII, and purified via agarose gel electrophoresis. The insert was digested with the restriction-endonuclease BamHI, and the resulting BamHI-HindIII fur DNA fragment was cloned in pUR290. This resulted in pC038. pC034 was similarly made from the insert of pMJ109 (cloning in pUR291). Now, instead of BamHI the restriction-endonuclease PstI was used. Reference for pUR vectors: EMBO J. 2, 1791-1794 (1983).

pMJ109 was made by cloning the insert of pMJ109 in the HindIII site of pATH1.

References for pATH1: Nucl. Acids Res. 9, 6647-6668 (1981)

J. Virol. 49, 132-141 (1984).

The host for the constructs in the pUR vectors is JM109 (Gene 33, 103-119 (1985)). This host is an E. coli derivative. The host for the construct in pATH1 is the E. coli strain RR1 (Gene 2, 95 (1977)).

Synthesis of the fusion proteins by means of the pUR vectors was effected by growing the bacteria in 2xSOB medium, to which isopropyl-$\beta$-D-thiogalactopyranoside had been added.

Synthesis by means of the pATH1 vector was effected by growing the bacteria in tryptophan-free M9 medium, to which 3-$\beta$-2-indolacrylic acid had been added.

For details of media, see Maniatis et al., Molecular Cloning: A Laboratory Manual, pp 440-441, Cold Spring Harbor Laboratory, 1982.

Preparation of monoclonal antibodies

Immunisation scheme (mice):

Day 0: 50 µg fusion protein in Freund complete adjuvant; subcutaneous.
Day 131: 100 µg fusion protein in incomplete Freund adjuvant; subcutaneous + intraperitoneal.
Day 179: 200 µg fusion protein in phosphate buffer/NaCl; intravenous.
Day 180: 200 µg fusion protein in phosphate buffer/NaCl; intravenous.
Day 181: 200 µg fusion protein in phosphate buffer/NaCl; intravenous.
Day 183: fusion.

As the fusion partner for the spleen cells, myeloma SP2/0 (Nature 276, 269-270 (1978) was used.

During the fusion, standard procedures (Kohler and Milstein) were used and standard medium. The foetal calf serum used was selected for its capacity to grow hybridomas.

Description of the drawings

In Fig. 1, human and feline DNA upstream of the v-fes/fps homologous region are compared.

Part A diagrammatically shows the cellular homologue of v-fes/fps and its upstream region in man and cat. In the middle of part A, the upstream regions are depicted in greater detail. Heavy bars designate the DNA regions in which man and cat have genetic sequences in common. Recognition sequences for restriction endonucleases are coded as follows:

B = BamHI
E = EcoRI
Hp = HpaI.
K = KpnI
P = PstI
Xb = XbaI
Xh = XHoI.

Part B gives an interpretative picture of the heteroduplex between the 9.2 kb human HpaI/EcoRI DNA fragment and the 14 kb feline EcoRI/EcoRI DNA fragment, which interpretative picture is based on the results of electron micrography (not shown).

Part C gives a diagrammatic picture of the results of measurements on 25 heteroduplexes. The loops shown are observed in all heteroduplexes. Arrows point to the positions and frequencies of loops found in a part of the heteroduplex structures.

Fig. 2 shows probes 1, 2 and 3 used for hybridization analysis of poly(A)-selected mRNA isolated from KG-1 cells, and after glyoxalation and electrophoretic separation on a 1 % agarose gel immobilized on Hybond N.

Fig. 3 shows the nucleotide sequence of the 0.95 kb fes/fps -related cDNA together with the amino acid sequence based thereon, in the conventional one-letter code. The consensus sequence for the polyadenylation signal is underlined. The poly(A) stretch of the cDNA clone is designated by $(A)_n$. Arrows point to the positions of the three possible poly(A) addition sites. The open box contains the possible phospho acceptor tyrosine. The asterisk indicates the stop codon.

Fig. 4 shows the nucleotide sequence of the 5′ portion of the human cellular homologue of v-fes/fps and the region immediately upstream thereof. The nucleotide sequence of the 4.16 kb human Bam HI/BglII DNA fragment comprises fur exon Z and c-fes/fps exons 1 and 2. Proven exon sequences are indicated by capitals. Proven exon boundaries are indicated by arrows. Relevant data of the nucleotide sequence of feline genomic DNA are stated in square brackets below the corresponding sequences in the human genome. The consensus sequence for the polyadenylation signal of the fur transcription unit is underlined. The open box represents a potential CATT box. A putative start codon is identified by a solid triangle.

Fig. 5 diagrammatically shows the topography of the human v-fes/fps cellular homologue and the region immediately upstream thereof. At the top, there is shown a diagrammatic restriction map of a 22 kb human DNA region comprising the v-fes/fps cellular homologue and its flanking sequences. The numbered black boxes represent the c-fes/fps exons described before. The open box representing exon 1 is dotted because the 5′ boundary is as yet undetermined. The solid triangle above exon 2 indicates the position of the putative start codon. The asterisk above exon 19 indicates the position of a TGA stop codon. The non-coding region of exon 19 is designated by an open box.

The hatched box Z represents sequences of the 3′ region of the fur transcription unit.

At the bottom, the positions of the genetic sequences in the cDNA clones are shown. The positions of consensus sequences for polyadenylation signals are designated by arrows.

Fig. 6 shows the topography of the human fur transcription unit. At the top, the position of fur relative to the c-fes/fps proto-oncogene, as based on homology in the upstream regions of feline and human c-fes/fps proto-oncogenes, is indicated by a solid bar. At the bottom, there is shown a detailed restriction map of the human fur transcription unit. Exons S to Z are shown in boxes. The polyadenylation signal is indicated by an arrow. The asterisk shows the position of the stop codon. The shortened code of restriction endonuclease recognition sequences is similar to that in Fig. 1; Sm = SmaI.

Fig. 7 shows the nucleotide sequence of a 5.4 kb fragment from the Sau 3A site to the EcoRI site in the fur genomic clone. The positions of the exons, as based on a comparison with the 3.1 kb cDNA sequence, are indicated by arrows. Exon sequences are designated by capitals. The possible 5′ ends of exon S are underlined. The polyadenylation signal is indicated by a dotted line. The asterisk indicates the stop codon. The solid triangle indicates the position of the polyadenylation site.

Fig. 8 shows an analysis of the open reading frames in the 3.1 kb fur cDNA nucleotide sequence. Vertical bars represent stop codons.

In Fig. 9, part A, the partial amino acid sequence is shown, derived from the large open reading frame (1498 bp) in the 3.1 kb fur cDNA nucleotide sequence. The cystein-rich region (CRR) is underlined, potential N-glycosylation sites are shown in a box, and the position of the transmembrane domain (TM) is indicated by a dotted line. The domain last mentioned is based on the hydrophobicity analysis shown in part B. Part C compares the transmembrane domains of furin and class II MHC antigens; vertical lines indicate homology, while conservative amino acid substitutions are given by a colon.

In Fig. 10, the cystein-rich regions of furin, the human insulin receptor (HIR) and the human epidermal growth factor receptor (HER) are compared. Homology and conservative amino acid substitutions are respectively designated by vertical lines and colons. Conserved cystein residues are shown in boxes.

TABLE A

| lung tissue | numbers of specimens tested | levels of fur expression | | |
|---|---|---|---|---|
| | | ++++ | ++ | control |
| SCLC | 7 | 0 | 1 | 6 |
| squamous cell lung carcinomas | 14 | 13 | 0 | 1 |
| adenocarcinomas | 16 | 16 | 0 | 0 |
| normal lung tissue | 5 | 0 | 0 | 5 |

## Claims

1. Recombinant DNA comprising the fur gene located in the genome upstream of the fes/fps proto-oncogene, or a significant part thereof, and optionally provided with a detectable label.

2. Isolated messenger RNA (mRNA) of the fur gene, obtainable from cells in which the fur gene is expressed by hybridization with fur DNA probes, optionally provided with a detectable label.

3. Recombinant cDNA of the fur gene obtainable by reverse transcription of fur mRNA, optionally provided with a detectable label.

4. Isolated protein called furin, being the translational product of the fur gene and obtainable by in vitroor in vivo translation of fur DNA or by isolation of the protein from cells in which the fur gene is expressed.

5. Monoclonal or polyclonal antibodies having a specificity for furin.

6. A method of detecting tumor cells in human and animal tissues or body fluids, such as sputum, urine, and the like, by hybridization assays with labelled RNA or DNA probes of the fur gene, or by immuno analytic methods, such as immunofluorescence ELISA, Western blotting, and immunoprecipitation/polyacrylamide gel electrophoresis, using furin-specific monoclonal or polyclonal antibodies.

FIG.1A

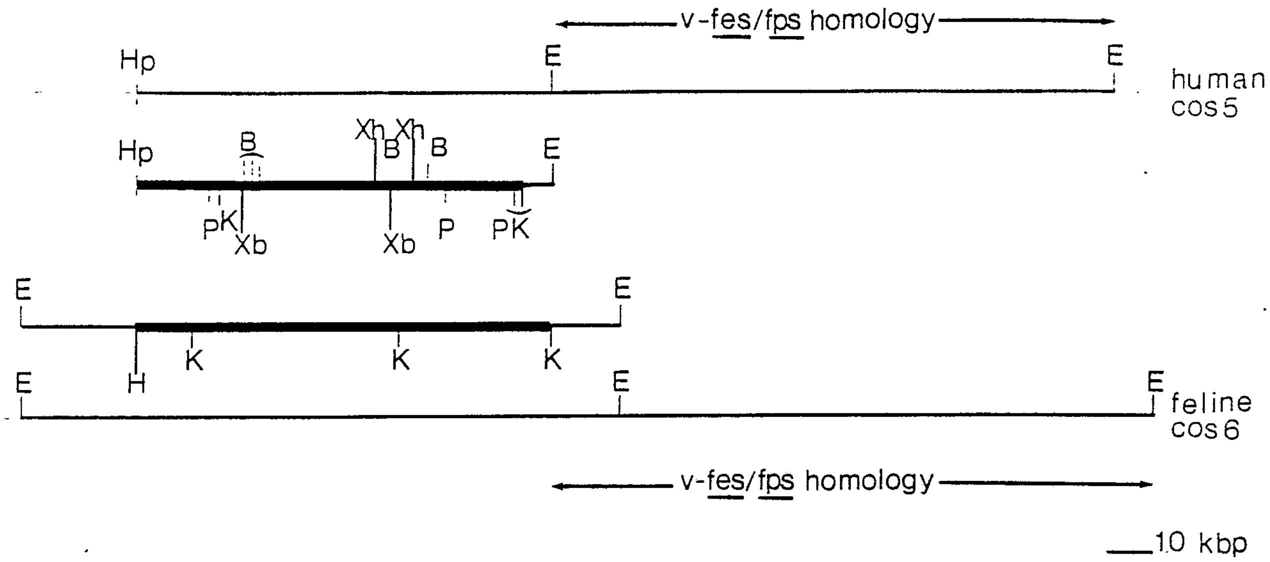


FIG.1B

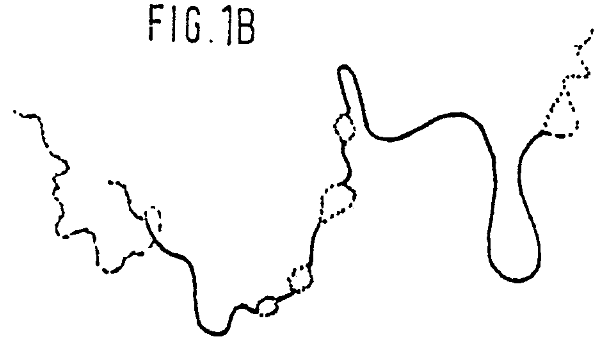

FIG.1C

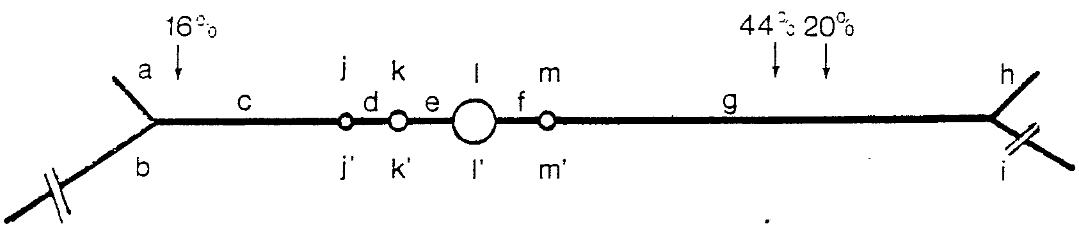


| | | |
|---|---|---|
| a : 0.61 ± 0.14 | f : 0.31 ± 0.05 | j=j' : 0.22 ± 0.07 |
| b : 2.32 ± 0.52 | g : 4.06 ± 0.50 | k=k' : 0.27 ± 0.07 |
| c : 1.69 ± 0.21 | h : 0.43 ± 0.19 | l : 0.43 ± 0.06 |
| d : 0.21 ± 0.05 | i : 1.65 ± 0.12 | l' : 0.55 ± 0.08 |
| e : 0.34 ± 0.06 | | m=m' : 0.22 ± 0.05 |

FIG.3

```
 k  q  y  s  h  p  n  i  v  r  l  i  g  v  c  t  q  k  q  p  i  y  i  v  m  e  l  v  q  g  g  d  f  l  t  f  l  r  t
TGAAGCAGTACAGCCACCCCAACATCGTGCGTCTCATTGGTGTCTGCACCCAGAAGCAGCCCATCTACATCGTCATGGAGCTTGTGCAGGGGGGCGACTTCCTGACCTTCCTCCGCACGG 120

e  g  a  r  l  r  v  k  t  l  l  q  m  v  g  d  a  a  a  g  m  e  y  l  e  s  k  c  c  i  h  r  d  l  a  a  r  n  c  l
AGGGGGCCCGCCTGCGGGTGAAGACTCTGCTGCAGATGGTGGGGGATGCAGCTGCTGGCATGGAGTACCTGGAGAGCAAGTGCTGCATCCACCGGGACCTGGCTGCTCGGAACTGCCTGG 240

v  t  e  k  n  v  l  k  i  s  d  f  g  m  s  r  e  e  a  d  g  v [y] a  a  s  g  g  l  r  q  v  p  v  k  w  t  a  p  e
TGACAGAGAAGAATGTCCTGAAGATCAGTGACTTTGGGATGTCCCGAGAGGAAGCCGATGGGGTCTATGCAGCCTCAGGGGGCCTCAGACAAGTCCCCGTGAAGTGGACCGCACCTGAGG 360

n  l  n  y  g  r  y  s  s  e  s  d  v  w  s  f  g  i  l  l  w  e  t  f  s  l  g  a  s  p  y  p  n  l  s  n  q  q  t  r
CCCTTAACTACGGCCGCTACTCCTCCGAAAGCGACGTGTGGAGCTTTGGCATCTTGCTCTGGGAGACCTTCAGCCTGGGGGCCTCCCCCTATCCCAACCTCAGCAATCAGCAGACACGGG 480

e  f  v  e  k  g  g  r  l  p  c  p  e  l  c  p  d  a  v  f  r  l  m  e  q  c  w  a  y  e  p  g  q  r  p  s  f  s  t  i
AGTTTGTGGAGAAGGGGGGCCGTCTGCCCTGCCCAGAGCTGTGTCCTGATGCCGTGTTCAGGCTCATGGAGCAGTGCTGGGCCTATGAGCCTGGGCAGCGGCCCAGCTTCAGCACCATCT 600

y  q  e  l  q  s  i  r  k  r  h  r  *
ACCAGGAGCTGCAGAGCATCCGAAAGCGGCATCGGTGAGGCTGGGACCCCCTTCTCAAGCTGGTGGCCTCTGCAGGCCTAGGTGCAGCTCCTCAGCGGCTCCAGCTCATATGCTGACAGC 720

TCTTCACAGTCCTGGACTCCTGCCACCAGCATCCACACTGCCGGCAGGATGCAGCGCCGTGTCCTCTCTGTGTCCCTGCTGCTGCCAGGGCTTCCTCTTCCGGGCAGAAACAATAAAACC 840
           |||
ACTTGTGCCCACTGAA(A)n                                                                                                    856
```

FIG.5

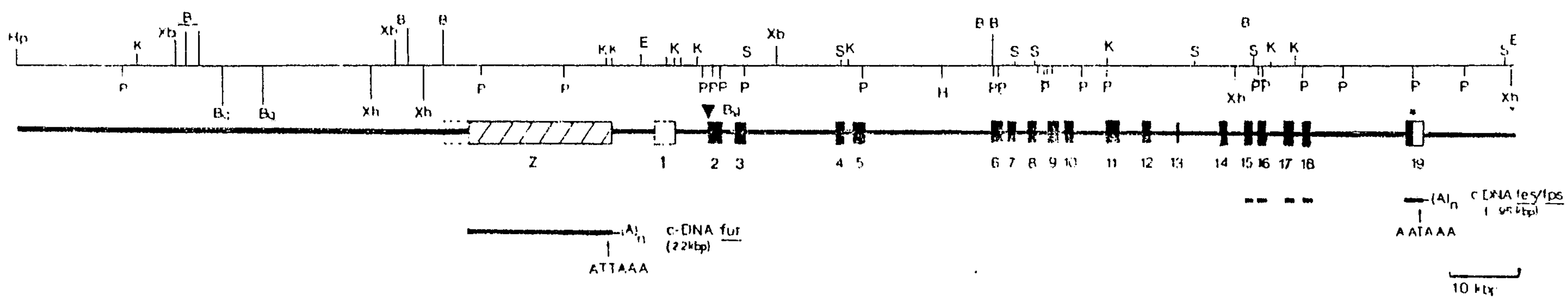

FIG.4I

BamHI

ggatcctggggatgtggtgacttggcttggggctgctgtggtcctggggctacagtctgtttagctgacacacacttgccctctctcccacgccggcagtgtgcgaggaaggcttctccc 120

tgcaccagaagagctgtgtccagcactgccctccaggcttcgcccccccaagtcctcgatacgcactatagcaccgagaatgacgtggagaccatccgggccagcgtctgcgccccctgcc 240

-> start c-DNA fur

acgcctcatgtgccacatgccaggggccggccctgacagaCTGCCTCAGCTGCCCCAGCCACGCCTCCTTGGACCCTGTGGAGCAGACTTGCTCCCGGCAAAGCCAGAGCAGCCGAGAGT 360

CCCCGCCACAGCAGCAGCCACCTCGGCTGCCCCCGGAGGTGGAGGCGGGGCAACGGCTGCGGGCAGGGCTGCTGCCCTCACACCTGCCTGAGGTGGTGGCCCGGCCTCAGCTGCGCCTTCA 480

TCGTGCTGGTCTTCGTCACTGTCTTCCTGGTCCTGCAGCTGCGCTCTGGCTTTAGTTTTCGGGGGGTGAAGGTGTACACCATGGACCGTGGCCTCATCTCCTACAAGGGGCTGCCCCCTG 600

AAGCCTGGCAGGAGGAGTGCCCGTCTGACTCAGAAGAGGACGAGGGCCGGGGCGAGAGGACCGCCTTTATCAAAGACCAGAGCGCCCTCTGATGAGCCCACTGCCCACCCCCTCAAGCCA 720

ATCCCCTCCTTGGGCACTTTTTAATTCACCAAAGTATTTTTTTATCTTGGGACTGGGTTTGGACCCCAGCTGGGAGGCAAGAGGGGTGGAGACTGTTTCCCATCCTACCCTCGGGCCCAC 840

CTGGCCACCTGAGGTGGGCCCAGGACCAGCTGGGGCGTGGGGAGGGCCGTACCCCACCCTCAGCACCCCTTCCATGTGGAGAAAGGAGTGAAACCTTTAGGGCAGCTTGCCCCGGCCCCG 960

GCCCCAGCCAGAGTTCCTGCGGAGTGAAGAGGGGCAGCCCTTGCTTGTTGGGATTCCTGACCCAGGCCGCAGCTCTTGCCCTTCCCTGTCCCTCTAAAGCAATAATGGTCCCATCCAGGC 1080

AGTCGGGGGCTGCCCTAGGAGATATCTGAGGGAGGAGGCCACCTCTCCAAGGGCTTCTGCACCCTCCACCCTGTCCCCCAGCTCTGGTGAGTCTTGGCGGCAGCAGCCATCATAGGAAGG 1200

GACCAAGGCAAGGCAGGTGCCTCCAGGTGTGCACGTGGCATGTGGCCTGTGGCCTGTGTCCCATGACCCACCCCTGTGCTCCGTGCCTCCACCACCACTGGCCACCAGGCTGGCGCAGCC 1320

AAGGCCGAAGCTCTGGCTGAACCCTGTGCTGGTGTCCTGACCACCCTCCCCTCTCTTGCACCCGCCTCTCCCGTCAGGGCCCAAGTCCCTGTTTTCTGAGCCCGGGCTGCCTGGGCTGTT 1440

GGCACTCACAGACCTGGAGCCCCTGGGTGGGTGGTGGGGAGGGGCGCTGGCCCAGCCGGCCTCTCTGGCCTCCCACCCGATGCTGCTTTCCCCTGTGGGGATCTCAGGGGCTGTTTGAGG 1560

ATATATTTTCACTTTGTGATTATTTCACTTTAGATGCTGATGATTTGTTTTTGTATTTTTAATGGGGGTAGCAGCTGGACTACCCACGTTCTCACACCCACCGTCCGCCCTGCTCCTCCC 1680

TGGCTGCCCTGGCCCTGAGGTGTGGGGGCTGCAGCATGTTGCTGAGGAGTGAGGAATAGTTGAGCCCCAAGTCCTGAAGAGGCGGGCCAGCCAGGCGGGCTCAAGGAAAGGGGGTCCCAG 1800

TGGGAGGGGCAGGCTGACATCTGTGTTTCAAGTGGGGCTCGCCATGCCGGGGGTTCATAGGTCACTGGCTCTCCAAGTGCCAGAGGTGGGCAGGTGGTGGCACTGAGCCCCCCCAACACT 1920

GTGCCCTGGTGGAGAAAGCACTGACCTGTCATGCCCCCCTCAAACCTCCTCTTCTGACGTGCCTTTTGCACCCCTCCCATTAGGACAATCAGTCCCCTCCCATCTGGGAGTCCCCTTTTC 2040

TTTTCTACCCTAGCCATTCCTGGTACCCAGCCATCTGCCCAGGGGTGCCCCCTCCTCTCCCATCCCCCTGCCCTCGTGGCCAGCCCGGCTGGTTTTGTAAGATACTGGGTTGGTGCACAG 2160

exZ <-

TGATTTTTTTCTTGTAATTTAAACAGGCCCAGCATTGCTGGTTCTATTTAATGGACATGAGATAATGTTAGAGGTTTTAAAGTGATTAAACGTGCAGACTATGCAAACCAGgcccagtct 2280

# FIG 4 II

```
ccagtgtggtaccgttgctcctgcatcgcagctgaggatagggggccagttaggcctacacagtggcctgcctgcctggatgtgggcccaagtcagaaggccaaagtcctccaaggggcg 2400

ggaggatgcgccagcccctagtggaggagctggtgcccctggggtggggctggtgacccctggtcctcaggagctgagcactaaactcccaaagtcctggtttccagcagtgtgaagaac 2520

tgggcctattgtgtcttcctgggctgaagtgatctggtcgccacaggctatagggctgaggcctaaggtggagggaggcctgactgaatcaagatgacttcttgtggggagcctgagtcc 2640

caaatggaaaactccacgcctgtccgctccccaacccctgccccttgatttccccaggtctcccttgggacaggaagcccctgcctgggggtaggaggatggggacaaaccactaggat 2760

ctgtatccgagaagcagtctctgttcgggatatttacttggaaattttattcaaatggaagctggcgcctgagcctctccttagggaattccgtgaggtggggagggctgggaccagggt 2880

tccctctttctcttctgcggtggccctggcctggtgctaggactgcgcgcctcccctcagtacccgcggacaccctgggcttccctgggcccagcatctgcctggggcctcgcctgggct 3000

ccccctcctgacccccaccttgcgccccttcccggtgttcccggggcgctgccgggccctggggcctgcggggcgcgggcggctcttggctg[gccattct]ttcccggccccctcctccc 3120

ttccgtttccgtggccgtgcggccggctagaggctgcggcccagcgcggagcaggggggctggcaggcgtcgggacggtcgggccggtcccgcccgccccttcccctccacaggcccgcc 3240
                                                                                                              ex 1 <-
ccggggcctgggccaactgaaaccgcgggaggaggaagcgcggaatcaggaactggccggggtccgcaccgggcctgagtcggtccgaggccgtcccaggagcaGCTGCCCGTCCGGgta 3360
                                                                                                         :::::::: :: ::::
                                                                                                        [GCTGCCCGCGC Ggta

cctctagccccggggcctggaggagcggtgggagctggggcgcggcaggcaggggcagagcaggcgttccgagggccagagacccacccaggtcggggtaggggccgcggaagggcggg 3480
 :::  :::: : :::   :
actcctgccctgaggctcg]

gatggccgcaggggcagggctcaggctgtgggcgcctgaggcttcagctggggcaggcttggcctgtcgaggacctgggcaagggtgtccctgtaaggggtggtgggtggaagggcctgg 3600

ggagggaggctccaggttggctcctgttcccgaacgtgcggaggagaccctgacgctaaggaagcaatgagggccagtccccaggccaggctgctgctgggtacccatggctgcgtgtga 3720

gcgaggcaggacccccacctcctccccgtctgcagtccatcctgaccctacagtccccagtctcctcgtcccatgcctccgtctccagctgctgccttgcctccagggatggcccctttc 3840
                                                                                                         :       ::  : ::
                                                                                                        [gccctctcctctctc
        -> ex2    ▼
tgtccccagAACAGCACTATGGCTTCTCTTCCGAGCTGTGCAGCCCCCAGGGCCACGGGGTCCTGCAGCAAATGCAGGAGGCCGAGCTTCGTCTACTGGAGGGCATGAGAAAGTGGATG 3960
::::::::: :: ::::::::::::::
tgtccccagGAGGGCACTATGGCTTC]
                                                                                                              ex2 <-
GCCCAGCGGGTCAAGAGTGACAGGGAGTATGCAGGACTGCTTCACCACATGTCCCTGCAGGACAGTGGGGGCCAGAGCCGGGCCATCAGCCCTGACAGCCCCCATCAGTCAGgtgggtctc 4080

tatgggactctggtgggtgctggcgtatctgccttctccttcctctcctgggggccctctggggcagtggctggagatct 4160
                                                                       BglII
```

FIG.6

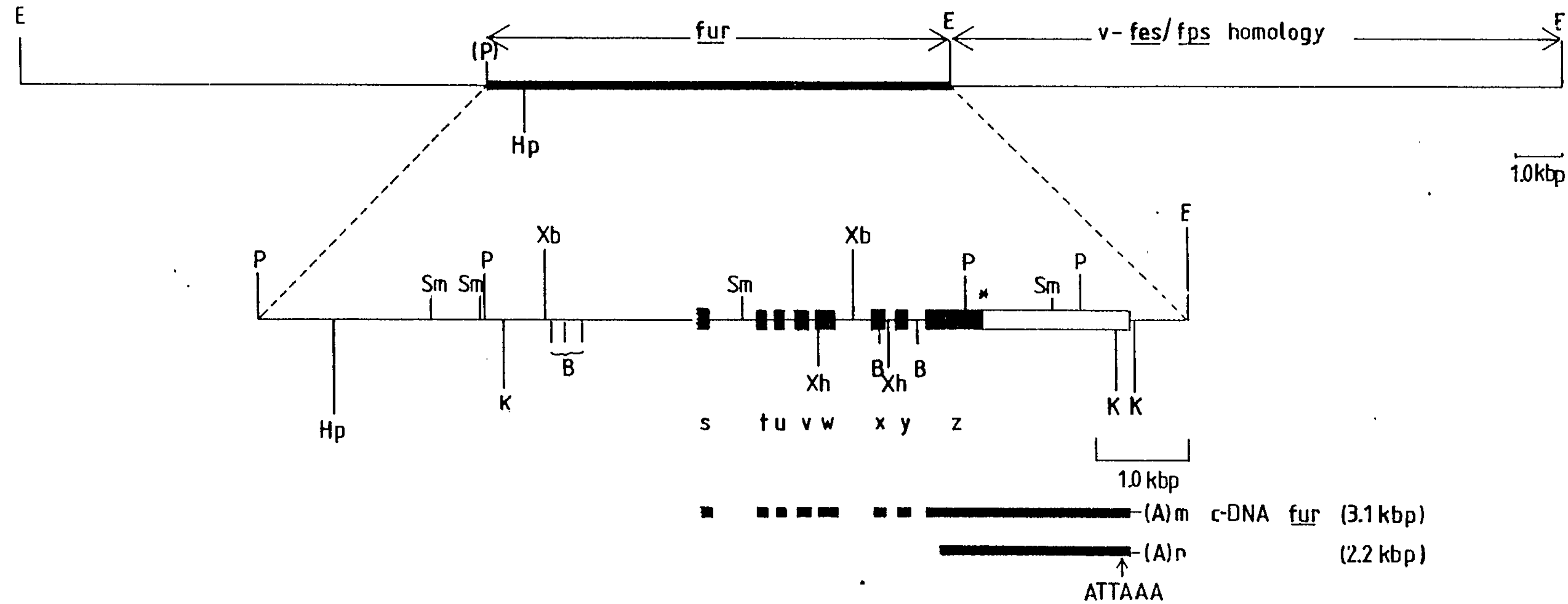
E
(P)
fur
E
v- fes/fps homology
E
Hp
1.0kbp
P
Sm
Sm
P
Xb
Xb
Sm
P
Sm
P
E
B
K
Hp
Xh
B
Xh
B
K K
s
t u v w
x y
z
1.0 kbp
(A)m
c-DNA fur (3.1 kbp)
(A)n
(2.2 kbp)
ATTAAA

FIG.8

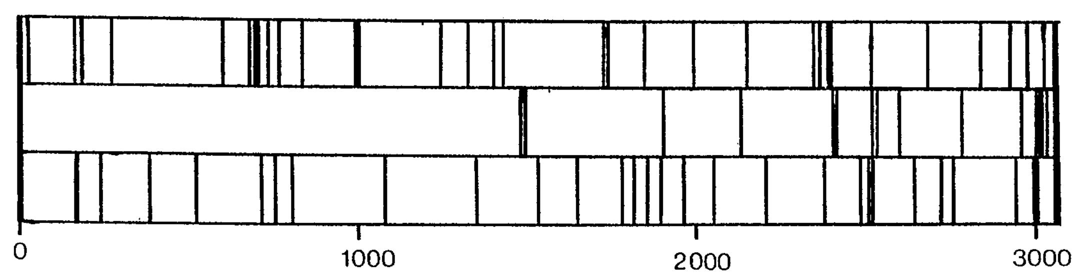
0
1000
2000
3000

# FIG. 7I

```
Sau3A
gatctcacaggggacaggaggttcccagagaatgaggctccagccttcccagttttccagcagtgtcctcctgccagccctccctcaagtcccaatcttgaatgaccccagcccactctg  120
                                    -> start c-DNA fur (3.1 kbp)
tccacagggccgagggggctgggctccatctttgtctgggcctcggggaaCGGGGCCGGGAACATGACAGCTGCAACTGCGACGGCTACACCAACAGTATCTACACGCTGTCCATCAG  240
                                                                                                   exS <-
CAGCGCCACGCAGTTTGGCAACGTGCCGTGGTACAGCGAGGCCTGCTCGTCCACACTGGCCACGACCTACAGCAGTGGCAACCAGAATGAGAAGCAGATCgtgagtcttacctgggggtg  360

ggggctggggagatggggctgctggctggctctgtccagcaccctcttttggaggctgtctgcctccacccccatgtggctgggtgatagtggctcaggcatcatgcaacatgaactctg  480
gggctctgtaggttcttggaggccatggagccatctctgaagactttgaaagcctagactctctctaaaacatactttgttcttattcatctccaccctctagttgaaccccttatt  600
cctttggaaaatgaggcccaggaggggctcgtcattagcatgtccacggcaagttagcaaggcagcagctgggacccggggttcttggccctggctccccataccatctgtggtgcccag  720
                                                                                              -> exT
ggcctgtatgcagagggagggtaggcaggtggctcctctagccccctccacccatcacaggccccaatatgattctcttcctggcagGTGACGACTGACTTGCGGCAGAAGTGCACGGAG  840
                                                                  exT <-
TCTCACACGGGCACCTCAGCCTCTGCCCCCTTAGCAGCCGGCATCATTGCTCTCACCCTGGAGGCCAAgtaagtgggtgggggccagcggcaaccctgtccctaccagcactctctgtag  960
                                                                               -> exU
ggcagccccttagggcagctggagagctgctcccaaaaaagactgatccccagcctctcccttccttctttgcagTAAGAACCTCACATGGCGGGACATGCAACACCTGGTGGTACAGACC  1080
                                                        exU <-
TCGAAGCCAGCCCACCTCAATGCCAACGACTGGGCCACCAATGGTGTGGGCCGGAAAGgtgagggcaggctggcccggcaggctggatgtggagttaggtagaaggcactctgtgcctga  1200
                                  -> exV
cagctgaccctaccttccctgtccccacagTGAGCCACTCATATGGCTACGGGCTTTTGGACGCAGGCGCCATGGTGGCCCTGGCCCAGAATTGGACCACAGTGGCCCCCCAGCGGAAGT  1320
                          exV <-
GCATCATCGACATCCTCACCGAGCCCAAgtgagggctggacccaggctgggaggggccagtgggacctgagagtcgcaggggggtgcccgctggtccctctgggccaggctgaccatcat  1440
                     -> exW
ggtgctctcctgcacagAGACATCGGGAAACGGCTCGAGGTGCGGAAGACCGTGACCGCGTGCCTGGGCGAGCCCAACCACATCACTCGGCTGGAGCACGCTCAGGCGCGGCTCACCCTG  1560
                                                                            exW <-
TCCTATAATCGCCGTGGCGACCTGGCCATCCACCTGGTCAGCCCCATGGGCACCCGCTCCACCCTGCTGGCAGCCAGgtgcttgctctgtccctgcccgccctgcccagcgccgcctcct  1680

ctcacagcccgcgtgcttgcctttgctcgctcacacggcccagggggcactgagtgctactcagtggggcactcttggcattttgggagggacaattttgtcctgtgggactgtcccatg  1800
ttgcaggaatccctggcttgcaaccatttaatgtcagtagtgtgccccaccttgtgcggtgcctgtgtacctttctagaatcccccagttgagcacgtctggctcactgagaaacagcca  1920
agccagcaggcacttctggccccatggggttggtctgcgtgggggaagggtgtgtggcccatgtgctgtgggttagatgtccctggcttggggtcccgcagaggcctcagggctgtgtgc  2040
                  -> exX
actcccctccccagGCCACATGACTACTCCGCAGATGGGTTTAATGACTGGGCCTTCATGACAACTCATTCCTGGGATGAGGATCCCTCTGGCGAGTGGGTCCTAGAGATTGAAAACACC  2160
                exX <-
AGCGAAGCCAACAACTATGgtactgggggcacttgaggggtaggggtacgaggtggagggctggcaggatctcgagcactgggtgtggtgccagcactgtcttaaactcttgcctcctcc  2280
                  -> exY
ccgctctggaacagGGACGCTGACCAAGTTCACCCTCGTACTCTATGGCACCGCCCCTGAGGGGCTGCCCGTACCTCCAGAAAGCAGTGGCTGCAAGACCCTCACGTCCAGTCAGGCCTG  2400
exY<-
TGTGGgtcagtagtgggtgctgttgggctttgggggcctgagtctgggggtaaggcgggtgcctgtcctgaagcccagctctaacagaaaaaagtctcaagagacctagggcccctgggg  2520

ctcttgggatgaccacagtcctggggctggaggatcctggggatgtggtgacttggcttggggctgctgtggtcctggggctacagtctgtttagctgacacacacttgccctctctccc  2640
              -> exZ
acgccggcagTGTGCGAGGAAGGCTTCTCCCTGCACCAGAAGAGCTGTGTCCAGCACTGCCCTCCAGGCTTCGCCCCCCAAGTCCTCGATACGCACTATAGCACCGAGAATGACGTGGAG  2760
```

FIG. 7 II

ACCATCCGGGCCAGCGTCTGCGCCCCCTGCCACGCCTCATGTGCCACATGCCAGGGGCCGGCCCTGACAGACTGCCTCAGCTGCCCCAGCCACGCCTCCTTGGACCCTGTGGAGCAGACT 2880

TGCTCCCGGCAAAGCCAGAGCAGCCGAGAGTCCCCGCCACAGCAGCAGCCACCTCGGCTGCCCCCGGAGGTGGAGGCGGGGCAACGGCTGCGGGCAGGGCTGCTGCCCTCACACCTGCCT 3000

GAGGTGGTGGCCGGCCTCAGCTGCGCCTTCATCGTGCTGGTCTTCGTCACTGTCTTCCTGGTCCTGCAGCTGCGCTCTGGCTTTAGTTTTCGGGGGGTGAAGGTGTACACCATGGACCGT 3120

GGCCTCATCTCCTACAAGGGGCTGCCCCCTGAAGCCTGGCAGGAGGAGTGCCCGTCTGACTCAGAAGAGGACGAGGGCCGGGGCGAGAGGACCGCCTTTATCAAAGACCAGAGCGCCCTC 3240

*
TGATGAGCCCACTGCCCACCCCCTCAAGCCAATCCCCTCCTTGGGCACTTTTTAATTCACCAAAGTATTTTTTTATCTTGGGACTGGGTTTGGACCCCAGCTGGGAGGCAAGAGGGGTGG 3360

AGACTGTTTCCCATCCTACCCTCGGGCCCACCTGGCCACCTGAGGTGGGCCCAGGACCAGCTGGGGCGTGGGGAGGGCCGTACCCCACCCTCAGCACCCCTTCCATGTGGAGAAAGGAGT 3480

GAAACCTTTAGGGCAGCTTGCCCCGGCCCCGGCCCCAGCCAGAGTTCCTGCGGAGTGAAGAGGGGCAGCCCTTGCTTGTTGGGATTCCTGACCCAGGCCGCAGCTCTTGCCCTTCCCTGT 3600

CCCTCTAAAGCAATAATGGTCCCATCCAGGCAGTCGGGGGCTGGCCTAGGAGATATCTGAGGGAGGAGGCCACCTCTCCAAGGGCTTCTGCACCCTCCACCCTGTCCCCCAGCTCTGGTG 3720

AGTCTTGGCGGCAGCAGCCATCATAGGAAGGGACCAAGGCAAGGCAGGTGCCTCCAGGTGTGCACGTGGCATGTGGCCTGTGGCCTGTGTCCCATGACCCACCCCTGTGCTCCGTGCCTC 3840

CACCACCACTGGCCACCAGGCTGGCGCAGCCAAGGCCGAAGCTCTGGCTGAACCCTGTGCTGGTGTCCTGACCACCCTCCCCTCTCTTGCACCCGCCTCTCCCGTCAGGGCCCAAGTCCC 3960

TGTTTTCTGAGCCCGGGCTGCCTGGGCTGTTGGCACTCACAGACCTGGAGCCCCTGGGTGGGTGGTGGGGAGGGGCGCTGGCCCAGCCGGCCTCTCTGGCCTCCCACCCGATGCTGCTTT 4080

CCCCTGTGGGGATCTCAGGGGCTGTTTGAGGATATATTTTCACTTTGTGATTATTTCACTTTAGATGCTGATGATTTGTTTTTGTATTTTTAATGGGGGTAGCAGCTGGACTACCCACGT 4200

TCTCACACCCACCGTCCGCCCTGCTCCTCCCTGGCTGCCCTGGCCCTGAGGTGTGGGGGCTGCAGCATGTTGCTGAGGAGTGAGGAATAGTTGAGCCCCAAGTCCTGAAGAGGCGGGCCA 4320

GCCAGGCGGGCTCAAGGAAAGGGGGTCCCAGTGGGAGGGGCAGGCTGACATCTGTGTTTCAAGTGGGGCTCGCCATGCCGGGGGTTCATAGGTCACTGGCTCTCCAAGTGCCAGAGGTGG 4440

GCAGGTGGTGGCACTGAGCCCCCCCAACACTGTGCCCTGGTGGAGAAAGCACTGACCTGTCATGCCCCCCTCAAACCTCCTCTTCTGACGTGCCTTTTGCACCCCTCCCATTAGGACAAT 4560

CAGTCCCCTCCCATCTGGGAGTCCCCTTTTCTTTTCTACCCTAGCCATTCCTGGTACCCAGCCATCTGCCCAGGGGTGCCCCCTCCTCTCCCATCCCCCTGCCCTCGTGGCCAGCCCGGC 4680

TGGTTTTGTAAGATACTGGGTTGGTGCACAGTGATTTTTTTCTTGTAATTTAAACAGGCCCAGCATTGCTGGTTCTATTTAATGGACATGAGATAATGTTAGAGGTTTTAAAGTGATTAA 4800
exZ <-
ACGTGCAGACTATGCAAACCAGgcccagtctccagtgtggtaccgttgctcctgcatcgcagctgaggatagggggccagttaggcctacacagtggcctgcctgcctggatgtgggccc 4920
▲

aagtcagaaggccaaagtcctccaaggggcgggaggatgcgccagcccctagtggaggagctggtgcccctggggtggggctggtgacccctggtcctcaggagctgagcactaaactcc 5040
caaagtcctggtttccagcagtgtgaagaactgggcctattgtgtcttcctgggctgaagtgatctggtcgccacaggctatagggctgaggcctaaggtggagggaggcctgactgaat 5160
caagatgacttcttgtggggagcctgagtcccaaatggaaaactccacgcctgtccgctccccaacccctgccccttgatttccccaggtctcccttgggacaggaagcccctgcctggg 5280
ggtaggaggatggggacaaaaccactaggatctgtatccgagaagcagtctctgttcgggatatttacttggaaattttattcaaatggaagctggcgcctgagcctctccttagggaat 5400
tc
EcoRI

# FIG.9A

```
        10         20         30         40         50         60         70         80         90        100        110        120
GGREHDSCNC DGYTNSIYTL SISSATQFGN VPWYSEACSS TLATTYSSGN QNEKQIVTTD LRQKCTESHT GTSASAPLAA GIIALTLEAN KNLTWRDMQH LVVQTSKPAH LNANDWATNG

       130        140        150        160        170        180        190        200        210        220        230        240
VGRKVSHSYG YGLLDAGAMV ALAQNWTTVA PQRKCIIDIL TEPKDIGKRL EVRKTVTACL GEPNHITRLE HAQARLTLSY NRRGDLAIHL VSPMGTRSTL LAARPHDYSA DGFNDWAFMT

       250        260        270        280        290        300        310        320        330        340        350        360
THSWDEDPSG EWVLEIENTS EANNYGTLTK FTLVLYGTAP EGLPVPPESS GCKTLTSSQA CVVCEEGFSL HQKSCVQHCP PGFAPQVLDT HYSTENDVET IRASVCAPCH ASCATCQGPA

       370        380        390        400        410        420        430        440        450        460        470        480
LTDCLSCPSH ASLDPVEQTC SRQSQSSRES PPQQQPPRLP PEVEAGQRLR AGLLPSHLPE VVAGLSCAFI VLVFVTVFLV LQLRSGFSFR GVKVYTMDRG LISYKGLPPE AWQEECPSDS

       490
EEDEGRGERT AFIKDQSAL
```

# FIG.10

```
furin  EANNYGTLTKFTLVLYG-TAPEGL-PVPPESSG-CKTLTSSQACVVCEEGFSLHQKSCVQHCPPG--FAPQVLDTHYSTE     335'
hir    THSHCQKVCPTICKSHGCTA-EGL-CCHSECLGNCSQPDDPTKCVACRN-FYLDGR-CVETCPPP--YY-HFQDWRCVNF     283''
her    QKLTKI-ICAQQC-SGRCRGKSPSDCCHNQCAAGCTGPRE-SDCLVCRK-FRDEAT-CKDTCPPLMLYN-PTTYQMDVNP     281'''
furin  EANNYGTLTKFTLVLYG-TAPEGL-PVPPE-SSGCKTLTSSQACVVCEEGFSLHQKSCVQHCPPG--FAPQVLDTHYSTE     335'

furin  NDVETIRASVCAPCHASC--ATCQGPALTDCLS-CPSH-ASLDPVE-QTCSRQSQSSRESPPQQQPPRLPPEVEAGQRLR     410'
hir    SFCQDLHHKCKNSRRQGC-HQYVI--HNNKCIPECPSG-YTMNSSN-LLCTPCLGPCPKVCHLLEGEKTIDSVTSAQELR     358''
her    EGKYSFGATCVK----KCPRNYVVTDH-GSCVRACGADSYEMEEDGVRKCKKCEGPCRKVCNGIGIGEFKDSLSINATNI     360'''
furin  NDVETIRASVCAPCHASC--ATCQGPALTDCLS-CPSH-ASLDPVE-QTCSRQSQSSRESPPQQQPPRLPPEVEAGQRLR     410'
```

FIG.2

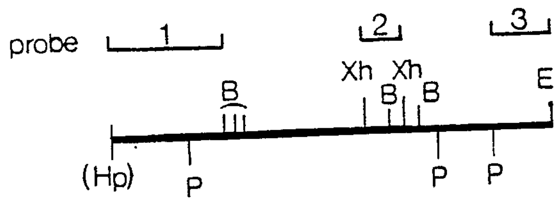


FIG.9B

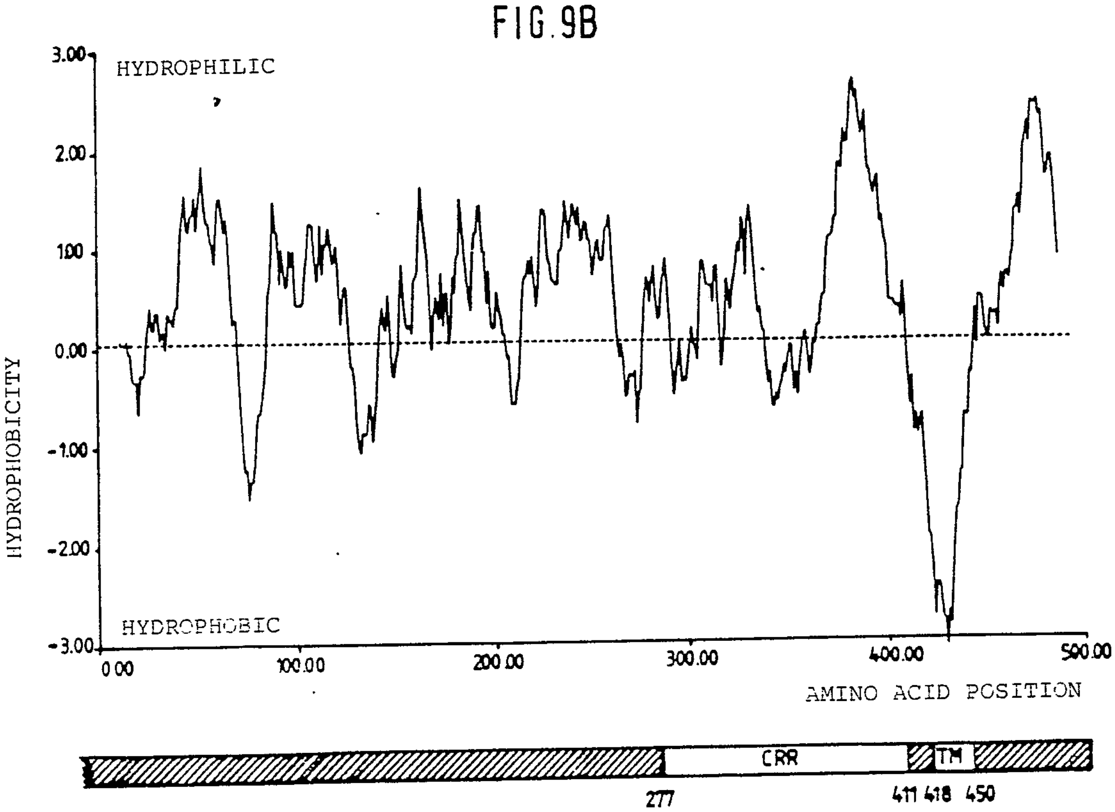


FIG.9C

```
         420         430         440         450
furin         LPEVVAGLSCAFIVLVFVTVFLVLQLRSGFSFR
                ..|||   . .|  |||..  |||    |
Class II MHC  TVVCALGLSVGLVGIVVGTVFIIRGLRSVGASR
          220         230         240         250
```

European Patent Office

# EUROPEAN SEARCH REPORT

Application number

EP 87 20 0940

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | THE EMBO JOURNAL, vol. 4, no. 11, 1985, pages 2897-2903, IRL Press Ltd, Oxford, GB; A.J.M. ROEBROEK et al.: "The structure of the human c-fes/fps proto-oncogene" * Whole article * | 1 | C 12 N 15/00<br>C 07 H 21/02<br>C 07 H 21/04<br>C 07 K 15/06<br>C 12 Q 1/68<br>G 01 N 33/53<br>G 01 N 33/577 |
| A | EP-A-0 108 564 (THE REGENTS OF THE UNIVERSITY OF CALIFORNIA) * Pages 6,7; page 1, lines 25-35; page 18, lines 29-31 * | 1-4,6 | |
| A,D | SCIENCE, vol. 216, 4th June 1982, pages 1136-1138, AAAS, US; J. GROFFEN et al.: "Isolation of human oncogene sequences (v-fes Homolog) from a cosmid library" * Whole article, especially figure 3 * | 1 | TECHNICAL FIELDS SEARCHED (Int. Cl.4)<br>C 12 N |
| X,P | MOLECULAR AND BIOLOGICAL REPORTS, vol. 11, no. 2, 11th June 1986, pages 117-125, Dr. W. Junk Publishers, Dordrecht, NL; A.J.M. ROEBROEK et al.: "Characterization of human c-fes/fps reveals a new transcription unit (fur) in the immediately upstream region of the proto-oncogene" * Whole article * | 1-3 | |

The present search report has been drawn up for all claims

| Place of search | Date of completion of the search | Examiner |
|---|---|---|
| THE HAGUE | 24-08-1987 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document